# EUROPEAN PATENT SPECIFICATION

(11) **EP 3 683 276 B1**
(45) Date of publication and mention of the grant of the patent: **15.11.2023**
(21) Application number: 19152377.8
(22) Date of filing: 17.01.2019
(51) Int. Cl.: C09B 31/043, D06P 1/06, C07C 309/47, D06P 1/39

(54) **NEW BISAZO ACID DYES**
NEUE BISAZO-SÄUREFARBSTOFFE
NOUVEAUX COLORANTS D'ACIDE BISAZO

(43) Date of publication of application: 22.07.2020
(73) Proprietor: Archroma IP GmbH, 4153 Reinach (CH)
(72) Inventor: Nusser, Rainer, 79395 Neuenburg am Rhein (DE)
(74) Representative: Wallinger Ricker Schlotter Tostmann

(56) References cited:
- EP-A1- 3 271 424
- US-A- 2 640 824

## Description

The invention relates to novel bisazo dyes, a process for the preparation thereof, and use thereof in a dyeing process or a printing process.

### BACKGROUND OF THE INVENTION

Bisazo dyes are known in the art. Bisazo dyes are a class of organic dyes that contain at least two azo groups as chromophoric groups. Bisazo dyes are used in trichromatic dying or printing especially on fibrous substrates made from cellulose or natural or synthetic polyamide. Trichromatic dyeing or printing can utilize all customary and known dyeing and printing processes, such as a continuous process, exhaustion process, foam dyeing process and ink-jet process. In the trichromatic dyeing process the composition of three individual dyes produces a desired hue. Commonly, the individual dye components are provided in yellow, red and blue.

WO 2010/130379 A1 discloses a bisazo dye bearing at least one anionic substituent selected from carboxy or sulpho group. The dye bears one naphtyleneol group in vicinity to each azo group. The dyes according to WO 2010/130379 A1 are in particular suitable for dyeing or printing of fibrous organic substrates in red and violet shades.

WO 2010/130384 A1 discloses a bisazo dye bearing at least one anionic substituent which can be sulpho group. The dye bears one pyridone group in vicinity to each azo group. The dyes according to WO 2010/130384 A1 are in particular suitable for dyeing or printing of fibrous organic substrates in yellow shades.

EP3271424 discloses trisazo acid dyes bearing at least one sulfonyl group. These dyes are in particular suitable for dyeing and printing fibrous organic substrates in blue shades.

There is an ongoing need for novel bisazo dyes suitable for blue dyeing, in particular suitable as a blue component in trichromatic dyeing/ printing which provides good properties such as dye levelness, color fastness and build-up behavior.

### OBJECT OF THE INVENTION

It has therefore been an object of the present invention to provide novel bisazo dyes that have improved properties, such as dye levelness (i.e. the uniformity of color shade along the substrate to be dyed), color fastness (light and wet/wash fastness, i.e. the resistance of the color to fading and running when exposed to light and wetness), and build-up behavior.

### SUMMARY OF THE INVENTION

The object is achieved by a bisazo dye according to the invention of the general formula (I) in its free acid, salt or mixed salt form. It has been surprisingly found that the compound of formula (I) has good dyeing characteristics, such as levelness, light and wet/washing fastnesses and build-up behavior, compared to the dyes or dye mixtures known in the prior art when applied to substrates, in particular when applied to substrates with a high surface like microfibers.

The improvement of compound of formula (I) is particularly achieved during dyeing and printing processes, such as inkjet printing and trichromatic dyeing/ printing.

In a first aspect the invention relates to a compound of formula (I) in its free acid, salt or mixed salt form 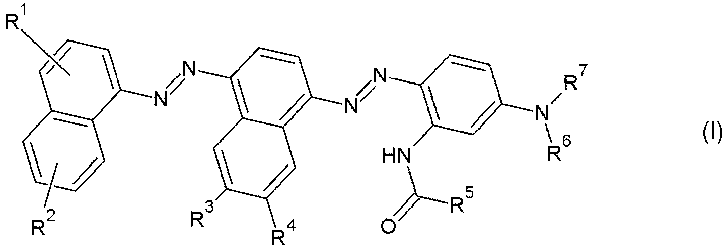 wherein
- R¹: signifies hydrogen or SO₃M,
- R²: signifies hydrogen or SO₃M,
- R³: signifies hydrogen or SO₃M,
- R⁴: signifies hydrogen or SO₃M,
- R⁵: signifies hydrogen, unsubstituted unbranched C₁-C₈-alkyl or unsubstituted branched C₃-C₈-alkyl, or substituted unbranched C₁-C₈-alkyl or substituted branched C₃-C₈-alkyl, substituted or unsubstituted phenyl, or unsubstituted unbranched C₁-C₁₂-alkoxy, or unsubstituted branched C₃-C₁₂-alkoxy, or substituted unbranched C₁-C₁₂-alkoxy, or substituted branched C₃-C₁₂-alkoxy, or NH₂,
- R⁶ and R⁷: signify independently from each other unsubstituted unbranched C₁-C₈-alkyl or unsubstituted branched C₃-C₈-alkyl, or substituted unbranched C₁-C₈ alkyl or substituted branched C₃-C₈-alkyl, or aryl, or -(CH₂)ₙ-aryl with n = 1, 2, 3 or 4, where the aryl radicals may be substituted;

wherein the substituents of the substituted aryl are selected from the group of -H, -SO₃M, wherein M signifies a counter cation as defined below, unsubstituted or substituted, linear or branched C1-6 alkyl, unsubstituted or substituted, linear or branched C1-6 alkoxy;
wherein the substituents of the substituted alkyl and alkoxy groups are selected from the group consisting of heteroatoms other than hydrogen, halogen, -CN, -NH₂ or -COOM, wherein M signifies a counter cation as defined below; or wherein the substituents in R5, R6 and R7 are selected from hydroxyl group or cyano group, or from hydroxyl group and cyano group under the proviso that said compound of formula (I) contains at least one anionic group, preferably carboxy or sulfo group, and at least one cation
   - M: selected from hydrogen cation, alkali metal cation, alkaline earth metal cation, ammonium ion and alkylammonium ion or mixtures thereof.

In a second aspect the invention relates to a process for preparing a compound of formula (I) wherein the process comprises providing a compound of formula (II) 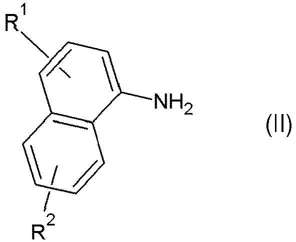 diazotizing the compound of formula (II) and coupling the diazotized compound of formula (II) onto the compound of formula (III), 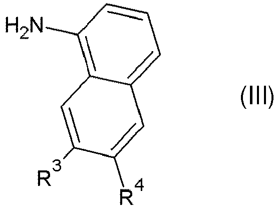 to obtain the resulting amine of formula (IV) 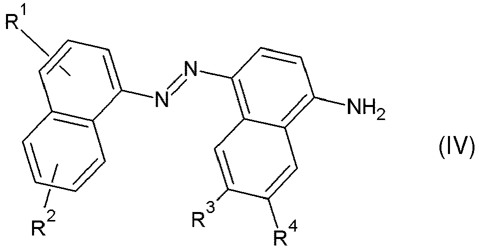 diazotizing the compound of formula (IV) and coupling the diazotized compound of formula (IV) onto compound of formula (V) 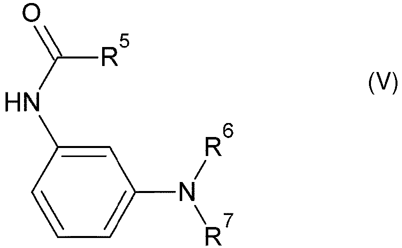 wherein the R¹ to R⁷ radicals are each as defined in at least one of claims 1 to 9.

In a third aspect the invention relates to the use of a compound of formula (I) as defined in at least one embodiment of the first aspect of the invention or prepared according to a process according to a third aspect of the invention in a dyeing process or a printing process, preferably a process for dyeing or printing a substrate.

In further embodiments of the third aspect, the invention in particular relates to the use of a compound as defined in formula (I), in a dyeing process or a printing process, preferably a process for dyeing or printing a substrate, in particular a fibrous substrate, preferably of fibrous material comprising or consisting of at least one natural polyamide, preferably selected from wool or silk, or at least one synthetic polyamide, preferably selected from nylon or mixtures thereof.

In a forth aspect the invention relates to a process for dyeing or printing a substrate in a trichromatic dyeing or trichromatic printing process for the manufacturing of a colored substrate, wherein the blue component used in the trichromatic dyeing or trichromatic printing process is a compound of formula (I) as defined in at least one embodiment of the firs aspect of the invention or prepared according to the second aspect of the invention.

In one embodiment, a compound as defined in formula (I), or a compound prepared according to the process for preparing a compound of formula (I) is used as blue component in a trichromatic dyeing or trichromatic printing process.

In a fifth aspect the invention relates to colored substrate comprising a compound according to at least one the embodiments of the first aspect of the invention or prepared according to ate least one of the embodiments of the second aspect of the invention.

### DETAILED DESCRIPTION OF THE INVENTION

In a first aspect the invention relates to a compound of formula (I) 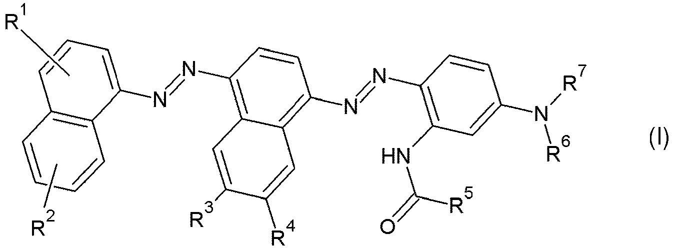 wherein
- R¹: signifies hydrogen or SO₃M,
- R²: signifies hydrogen or SO₃M,
- R³: signifies hydrogen or SO₃M,
- R⁴: signifies hydrogen or SO₃M,
- R⁵: signifies hydrogen, unsubstituted unbranched C₁-C₈-alkyl or unsubstituted branched C₃-C₈-alkyl, or substituted unbranched C₁-C₈-alkyl or substituted branched C₃-C₈-alkyl, substituted or unsubstituted phenyl, or unsubstituted unbranched C₁-C₁₂-alkoxy, or unsubstituted branched C₃-C₁₂-alkoxy, or substituted unbranched C₁-C₁₂-alkoxy, or substituted branched C₃-C₁₂-alkoxy, or NH₂,
- R⁶ and R⁷: signify independently from each other unsubstituted unbranched C₁-C₈-alkyl or unsubstituted branched C₃-C₈-alkyl, or substituted unbranched C₁-C₈ alkyl or substituted branched C₃-C₈-alkyl, or aryl, or -(CH₂)ₙ-aryl with n = 1, 2, 3 or 4, where the aryl radicals may be substituted;

wherein the substituents of the substituted aryl are selected from the group of -H, -SO₃M, wherein M signifies a counter cation as defined below, unsubstituted or substituted, linear or branched C1-6 alkyl, unsubstituted or substituted, linear or branched C1-6 alkoxy; wherein the substituents of the substituted alkyl and alkoxy groups are selected from the group consisting of heteroatoms other than hydrogen, halogen, -CN, -NH₂ or-COOM, wherein M signifies a counter cation as defined below; or wherein the substituents in R5, R6 and R7 are selected from hydroxyl group or cyano group, or from hydroxyl group and cyano group under the proviso that said compound of formula (I) contains at least one anionic group, preferably sulfo group, and at least one cation
- M: selected from hydrogen cation, alkali metal cation, earth alkaline metal cation, ammonium ion and alkylammonium ion or mixtures thereof.

The compound of formula (I) provide blue dyeings preferably on polyamide and wool fibers with very good light and wet fastness.

The term "*anionic group*" signifies a group capable of forming an anion and a respective counterion in the form of a cation. The SO₃M group may be termed as an anionic group. SO₃M group is capable of forming a SO₃⁻ group as an anion. Suitable counterions M⁺ are preferably H⁺, or alkali metal ions such as Na⁺ and K⁺ ions, or alkaline earth metal ions such as Ca²⁺ and Mg²⁺ ions, or ammonium cation, alkylammonium cation, e.g. mono-, di-, tri- and tetramethyl or mono-, di-, tri- and tetraethyl ammonium cations or mixtures thereof.

Within the context of the present application the terms "*SO₃H*" or "*SO₃*⁻" are regarded to correspond to the term "sulfo" group. Within the present application the terms "sulfo" is meant to represent equivalently the free acid, the salt and the mixed salt form.

Within the understanding of the present application the term "free acid" corresponds to an anionic group, wherein H⁺ forms the counter cation. An example for a free acid is SO₃H. Correspondingly, the bisazo dye of formula (I) may be termed to be in its free acid form, if the sulfo groups are in its free acid form.

If the counter cation M⁺ of the anionic group is alkali or alkali earth metal cation, or ammonium cation or alkylammonium cation the anionic group may be termed to be in its salt form. Correspondingly, the bisazo dye may be also termed to be in its salt form.

In case the counter cation M⁺ of the anionic group is formed by a mixture of different cations H⁺, alkali or alkali earth metal ion, or ammonium cation or alkylammonium cation, the anionic group and the bisazo dye of formula (I) itself may be termed to be in its mixed salt form.

The term "*alkali metal cation*" within the context of the present application encompasses the cations of group 1 elements of the periodic table except for hydrogen, i.e. Li⁺, Na⁺, K⁺, Rb⁺, Cs⁺ and Fr⁺. In a preferred embodiment of the present application, the term alkaline metal cation encompasses the cations of Li⁺, Na⁺ and K

The term "*alkaline earth metal cation*" within the context of the present application encompasses the cations of group 2 elements of the periodic table, i.e. Be²⁺, Mg²⁺, Ca²⁺, Sr²⁺, Ba²⁺ and Ra²⁺. In a preferred embodiment of the present application, the term alkaline earth metal cation encompasses the cations of Mg²⁺ and Ca²⁺.

The term "*alkylammonium cation*" within the context of the present application encompasses a positively charged nitrogen atom carrying four residues in total, wherein at least one residue is an alkyl residue having 1 to 10 carbon atoms and the remaining residues might be hydrogen or alkyl residue having 1 to 10 carbon atoms.

As used within the context of the present application, the term "*compound*" encompasses any single compound or any mixture of two or more compounds of formula (I) as herein defined. Thus, the term "*compound*" also encompasses mixtures of two or more compounds of formula (I) which are different with respect to their constitutional structure, their configurational structure and/ or with respect to the counter cations.

The compound of general formula (I) is built from an amino naphthalene unit which is coupled via diazotization to a further naphtylene ring and to an 3-N,N dialkylamino anilid.

Within the context of the present application the term "*aryl*" denotes a monocyclic or polycyclic residue derived from an aromatic hydrocarbon. The aryl residue might be unsubstituted denoting in the context of the present application that the aromatic hydrocarbon residue carries only hydrogen atoms. The aryl residue might be substituted denoting in the context of the present application that the aromatic hydrocarbon residue is substituted with heteroatoms others than hydrogen. Preferred embodiments of the aryl residue are phenyl and naphtylene group.

The term "*phenyl*" within the context of the present application denotes an aromatic residue derived from benzene. The phenyl residue might be unsubstituted denoting in the context of the present application that the aromatic hydrocarbon residue carries only hydrogen atoms, i.e. a residue of formula C₆H₅. The phenyl residue might be substituted denoting in the context of the present application that the aromatic hydrocarbon residue is substituted with heteroatoms others than hydrogen.

The term "*naphtylene*" within the context of the present application denotes an aromatic residue derived from naphthalene, i.e. a compound consisting of two condensed benzene rings. The naphthyl residue might be unsubstituted denoting in the context of the present application that the aromatic hydrocarbon residue carries only hydrogen atoms, i.e. a residue of formula C₁₀H₇. The naphthylene residue might be substituted denoting in the context of the present application that the aromatic hydrocarbon residue is substituted with heteroatoms others than hydrogen.

The substituents of the substituted aryl are selected from the group of -H, -SO₃M, wherein M signifies a counter cation as defined above, unsubstituted or substituted, linear or branched C₁₋₆ alkyl, unsubstituted or substituted, linear or branched C₁₋₆ alkoxy. Within the context of the present application the term "*substituted alkyl*" or "*substituted alkoxy*" denotes an alkyl residue or an alkoxy residue being substituted with heteroatoms others than hydrogen. The substituents of the substituted alkyl and alkoxy groups are selected from the group consisting of halogen, -CN, -NH₂ or -COOM, wherein M signifies a counter cation as defined above.

In further embodiments of the first aspect of the invention compound of formula (I) contains one, or two, or three or four SO₃M-groups, preferably the number of sulfo groups in compound of formula (I) equals 2.

In one embodiment of the first aspect of the invention in R⁵, R⁶, R⁷ methyl, ethyl, and propyl are preferred C₁-C₃ alkyl groups, wherein methyl and ethyl are particularly preferred.

In one further embodiment of the first aspect of the invention in R⁵ methoxy and ethoxy are preferred C₁-C₂ alkoxy groups, wherein methoxy is particularly preferred.

In one further embodiment of the first aspect of the invention in R⁶, R⁷ aryl is unsubstituted or substituted phenyl or naphtylene group.

In one further embodiment R¹ is bound to the 4-position. The numbers at the naphtylene ring presented in the below shown formula (Ia) will be used to describe the substitution pattern.

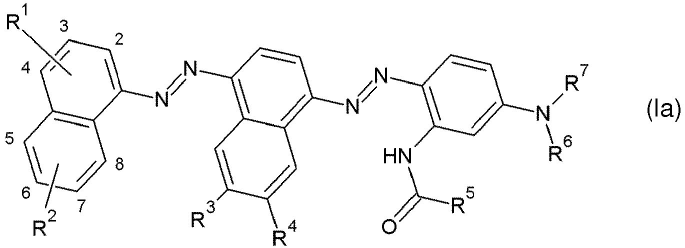

In one further embodiment of the first aspect of the invention R² is sulfo and is bound to the 5-, 6-, or 7-position.

In further embodiments of the first aspect of the invention the number of SO₃M groups equals two, wherein R¹ and R⁴, or R¹ and R³, or R² and R⁴, or R² and R³, signify SO₃M.

In further embodiments of the first aspect of the invention the number of SO₃M groups in compound of formula (I) equals two, wherein
R¹ and R⁴ signify SO₃M and R¹ is in the 4- position, or wherein
R¹ and R³ signify SO₃M and R¹ is in the 4- position, or wherein
R² and R⁴ signify SO₃M and R² is in the 7-position, or wherein
R² and R³ signify SO₃M and R² is in the 7-position, or wherein
R² and R⁴ signify SO₃M and R² is in the 5-position, or wherein
R² and R³ signify SO₃M and R² is in the 5-position, or wherein
R¹ and R² signify SO₃M and R¹ is in the 4-, and R² is in the 6-position, or wherein
R² and R⁴ signify SO₃M and R² is in the 6-position, or wherein
R² and R³ signify SO₃M and R² is in the 6-position.

In one further embodiment of the first aspect of the invention in compound of formula (I) R⁵ signifies unsubstituted unbranched C₁-C₈-alkyl. In one embodiment R⁵ signifies unsubstituted unbranched C₁-C₆-alkyl or may signify unsubstituted unbranched C₁-C₄-alkyl, or unsubstituted methyl, or unsubstituted ethyl, or unsubstituted unbranched propyl, or unsubstituted unbranched butyl, wherein unsubstituted methyl or ethyl are preferred

In one further embodiment of the first aspect of the invention in compound of formula (I) R⁶ and R⁷ signify independently from each other unsubstituted unbranched C₁-C₆-alkyl or unsubstituted branched C₃-C₄-alkyl or substituted unbranched C₁-C₆ alkyl or substituted branched C₃-C₄-alkyl. In particular, R⁶ and R⁷ may independently signify unsubstituted methyl, or unsubstituted ethyl, or unsubstituted unbranched propyl, or unsubstituted unbranched butyl, or unsubstituted unbranched pentyl, or unsubstituted unbranched hexyl, or substituted methyl, or substituted ethyl, or substituted unbranched propyl, or substituted unbranched butyl, or substituted unbranched pentyl, or substituted unbranched hexyl, or unsubstituted branched propyl, or unsubstituted branched butyl, or substituted branched propyl, or substituted branched butyl. R⁶ and R⁷ may signify the same substituents or may signify different substituents. In particular, R⁶ signifies unsubstituted unbranched C₁-C₆-alkyl and R⁷ signifies unsubstituted branched C₃-C₄-alkyl or substituted branched C₃-C₄-alkyl and vice versa. In an alternative, R⁶ signifies substituted unbranched C₁-C₆ alkyl and R⁷ signifies unsubstituted branched C₃-C₄-alkyl or substituted branched C₃-C₄-alkyl and vice versa, wherein the substituents are as defined above.

In one further embodiment wherein R⁵ signify substituted unbranched C₁-C₈-alkyl or substituted branched C₃-C₈-alkyl, substituted phenyl, or substituted unbranched C₁-C₁₂-alkoxy, or substituted branched C₃-C₁₂-alkoxy, the substituents in R⁵ signify -OH, -CN or NH₂.

In one further embodiment wherein R⁶ and R⁷ signify independently from each other substituted unbranched C₁-C₈ alkyl or substituted branched C₃-C₈-alkyl, or aryl, or -(CH₂)ₙ-aryl with n = 1, 2, 3 or 4, wherein the aryl radical may be substituted, the substituents in R⁶ and R⁷ signify -OH, -CN or NH₂. In certain embodiments R⁶ and R⁷ may signify independently from each other cyanoethyl or hydroxyethyl.

The compounds of the above-defined formula (I) may be prepared by suitable diazotation and coupling reactions. Diazotization and coupling conditions can be chosen by analogy with customary diazotization and coupling reactions.

A second aspect of the invention therefore relates to a process for preparing a compound of formula (I) wherein the process comprises
providing a compound of formula (II) 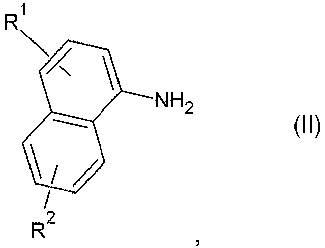
diazotizing the compound of formula (II) and coupling the diazotized compound of formula (II) onto compound of formula (III), 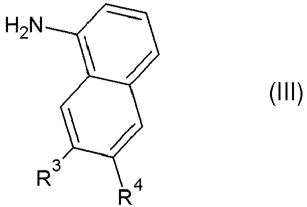
to obtain the resulting amine of formula (IV) 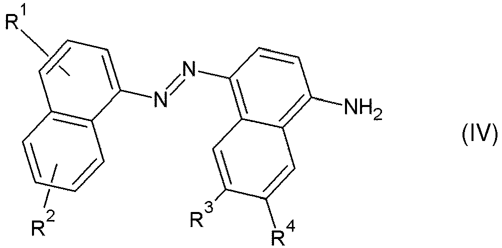
diazotizing the compound of formula (IV) and coupling the diazotized compound of formula (IV) onto compound of formula (V) 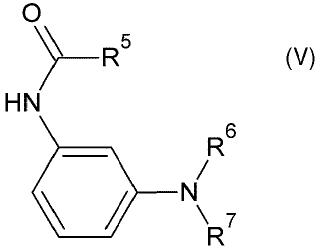
wherein the R¹ to R⁷ are each as defined in any of the embodiments according to the first aspect of the invention.

In one embodiment of the second aspect of the invention the diazotization is performed at -10°C to +10°C, preferably at -5°C to +10°C. Thereafter, the diazotized amine is allowed to react with the corresponding coupling component, preferably in aqueous solution.

The compound of formula (I) can be isolated from the reaction medium by conventional processes, for example by salting out with an alkali metal salt, filtering and drying, if appropriate under reduced pressure and at elevated temperature.

Depending on the reaction and/or isolation conditions, the compound of formula (I) can be obtained in its free acid, salt or as mixed salt form which contains, for example, one or more cations M⁺ selected from alkali metal cations, for example the sodium ion, or an ammonium ion or alkylammonium cation, for example mono-, di- or trimethyl- or - ethylammonium cations. The compound can be converted by conventional techniques from the free acid into a salt or into a mixed salt, or vice versa, or from one salt form into another. If desired, the compound of the formula (I) can be further purified by diafiltration, in which case undesired salts and synthesis by-products are separated off from the crude compound of formula (I).

The removal of undesired salts and synthesis by-products and partial removal of water from the crude solution of compound of formula (I) can be carried out by means of a semipermeable membrane by applying a pressure, whereby the compound of formula (I) is obtained without the undesired salts and synthesis by-products as a solution, and, if necessary, as a solid body in a conventional manner.

In a third aspect the invention therefore relates to the use of a compound of formula (I) as defined in any of the embodiments of the first aspect of the invention or prepared according to the second aspect of the invention in a dyeing process or a printing process, in particular a trichromatic dyeing/ printing process, preferably a process for dyeing or printing a substrate. The term "*substrate*" as used herein encompasses all substrates of natural or synthetic origin. The substrate may be present in the form of a textile, (i.e. material comprising or consisting of natural or synthetic polyamides such as wool, silk and all nylon types, cellulose or cotton), or in form of a plastic object. The term "substrate" also encompasses hydroxy- or nitrogen-containing materials.

In preferred embodiments the substrate is a plastic object, preferably a 3D-printed plastic object or a fibrous substrate, preferably a fibrous material comprising or consisting of at least one natural polyamide, preferably selected from wool or silk, or at least one synthetic polyamide, preferably selected from nylon or mixtures thereof.

The substrate to be dyed can be present in the form of yarn, woven fabric, loop-forming knitted fabric or carpet, for example. Fully fashioned dyeings are even perfectly possible on delicate substrates, examples being lamb-wool, cashmere, alpaca and mohair. The dyes of the invention are particularly useful for dyeing fine-denier fibres (microfibres).

Further examples for the form/appearance of the substrate are yarn, woven fabric, loop-formingly knitted fabric carpet comprising or consisting of an organic substrate, e.g. natural or synthetic polyamides (for example wool, silk and all nylon types), polyurethanes, cellulose as well as hydrophobic and non-absorbent substrates, for example plastics, metal and glass.

The substrates for dyeing can also be leather and fibrous materials, which comprise natural or synthetic polyamides and, particularly, natural or regenerated cellulose such as cotton, viscose and spun rayon.

In one embodiment, the substrates for dyeing are textiles comprising cotton.

Suitable substrates which can be dyed using compound of formula (I) in particular for printing are paper, plastic, textiles, metal, glass, or an overhead projector slide.

Suitable plastic objects which can be dyed using the compound of formula (I) can be formed by any traditional method known in the state of the art, like molding methods. Further, the plastic object can be formed by newly developed methods like 3D-printing methods. Commonly known 3D-printing methods are for example binder jetting, triple jetting (also referred to as PolyJet, MultiJet), stereolithography (SLA), digital light processing (DLP), multijet printing, fused deposition modelling (FDM), selective heat sintering (SHS), selective laser sintering (SLS), laminated object manufacturing (LOM), wax deposition modeling (WDM), 3-dimensional inkjet printing (3DP), thermoplastic extrusion, smooth curvature printing, selective deposition lamination, hybrid CNC, fused filament fabrication (FFF).

Dyeing is carried out as per known processes. The term "*exhaust dyeing process*" as used herein is to be understood as a process in which the dye is gradually transferred from a relatively large volume dyebath to the organic substrate which is thereby dyed over a relatively long period of time (see A Review of Textile Dyeing Processes, Perkins W. S, 1991. Textile Chemist & Colorist vol. 23(8) 23-27) Preference is given to dyeing in the exhaust process at a temperature of 30 to 140 °C. The dyeing process can be performed in temperature ranges of from 40 to 100°C, or 50 to 80°C, in other embodiments temperature ranges of from 80 to 120 °C, or from 80 to 100 °C may be used, depending on the thermal softening behavior of the plastic object. The liquor ratio is in the range from 3:1 to 40:1.

In a further embodiment the dyeing process can be a continuous dyeing process. The term "*continuous dyeing process*" as used herein is to be understood as a process in which the substrate to be dyed is fed continuously into a dye bath. Examples of a continuous dyeing process are pad-steam process or pad-dry process.

In one embodiment the compound of formula (I), in its free acid, salt or mixed salt form is particularly suitable for printing fibrous material consisting of natural or synthetic polyamide in violet to black shades. The compound of formula (I) and their salts are also suitable for producing ink-jet printing inks and for using these ink-jet printing inks to print substrates like plastic objects, in particular 3D-printed plastic objects or fibrous material which consists of natural or synthetic polyamide or cellulose (paper for example).

In a forth aspect the invention therefore relates to a process for dyeing or printing a substrate in a trichromatic dyeing or trichromatic printing process for the manufacturing of a colored substrate, wherein the blue or violet component used in the trichromatic dyeing or trichromatic printing process is a compound of formula (I) as defined in any of the embodiments of the first aspect of the invention, or prepared according to any of the embodiments of the second aspect of the invention.

In a fifth aspect the invention relates to a colored substrate comprising a compound of formula (I) according to any of the embodiments of the first aspect of the invention, or prepared according to any of the embodiments of the second aspect of the invention. In preferred embodiments the substrate is selected from cellulose, preferably paper, or natural polyamide, preferably wool or silk, or synthetic polyamide, preferably nylon.

The compound of formula (I) according to the present invention in its free acid, salt or mixed salt form is highly compatible with known acid dyes. Accordingly, the compound of formula (I) can be used alone in a dyeing or printing process or else as a component in a combination shade dyeing or printing composition together with other acid dyes of the same class, i.e. with acid dyes possessing comparable dyeing properties, such as for example fastness properties and exhaustion rates from the dye bath onto the substrate. The compound of formula (I) of the present invention can be used in particular together with certain other dyes having suitable chromophores. The obtained combination dyeings have similar fastness compared to dyeings with the individual dye. The ratio in which the dyes are present in a combination shade dyeing or printing composition is dictated by the hue to be obtained.

The compound of formula (I), as stated above, is very useful for dyeing natural and synthetic polyamides, i.e. wool, silk and all nylon types, and plastic objects, in particular 3D-printed plastic objects, on each of which dyeings having a high fastness level, especially good light fastness and good wet fastness (washing, alkaline perspiration) are obtained. The compound of formula (I) in its free acid, salt or mixed salt form has a high rate of exhaustion. The ability of the compound of formula (I) in tis free acid, its salt form or mixed salt form to build up is likewise very good. On-tone dyeings on the identified substrates are of outstanding quality. All dyeings moreover have a constant hue under artificial light. Furthermore, the fastness to decating and boiling is good.

One decisive advantage of the compounds of formula (I) according to the invention is that it is metal free and provides very homogeneous dyeings. Within the understanding of the present application the term "metal" refers to centered metals being bound by coordinative bindings to the dye compounds. Those dyes are referred to metal-complex dyes. However, within the meaning of the present application the term "metal free" does not extend to alkali or alkaline earth metal ions as exemplified throughout the present application.

The compound of formula (I) according to the invention can also be used as blue components in trichromatic dyeing or printing. Trichromatic dyeing or printing can utilize all customary and known dyeing and printing processes, such as, for example, the continuous process, exhaustion process, foam dyeing process and ink-jet process.

The blue component can, as described above, consist of a single component or of a mixture of different blue individual components conforming to formula (I). Preference is given to double and triple combinations.

The present invention is further illustrated by the following examples or preferred embodiments thereof. In the following examples, if not indicated otherwise, parts and percentages are by weight and temperatures are reported in degrees Celsius.

### EXAMPLES

### Preparation Example 1

111.6 parts of 1-aminonaphthalene-4-sulfonic acid were mixed with 750 parts of water. The pH of the resulting suspension was adjusted to 9.5 to 10 by adding of 0.5 parts by volume of 30% strength aqueous sodium hydroxide and stirred for a period of 30 minutes. 90.6 parts by volume of a 40% strength sodium nitrite solution were added at 20°C to 25 °C, in a dropwise manner within a period of 30 minutes. Then, the resulting suspension was added dropwise within 60 to 90 min to a beaker charged with 500 parts of water, 500 parts of ice and 127 parts by volume of approximately 30% strength aqueous hydrochloric acid. The temperature was maintained during the addition in the range of -5 °C to +10 °C by addition of ice. After the diazotization had ended, excess sodium nitrite was destroyed with amidosulfonic acid.

111.6 parts of 1-aminonaphthalene-6-sulfonic acid were suspended in 850 parts of water. The suspension was adjusted to a pH of 3 to 3.5 with hydrochloric acid. The diazo suspension was then added in the course of a period of 90 to 120 minutes with vigorous stirring. During the addition, the pH was maintained at about 3.5 by metered addition of sodium carbonate solution.

After the coupling reaction had ended, the resulting compound of the formula (VI) 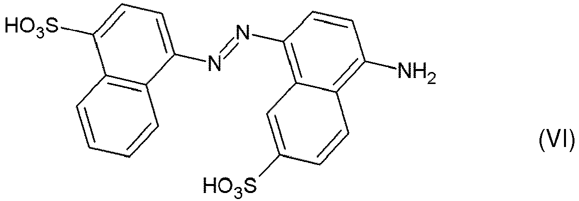 was salted out with sodium chloride, filtered off and dried at 50 °C under reduced pressure.

A suspension of 131.2 parts of 3-N,N-diethylamino-1-acetanilid in 400 parts of water was mixed with a diazonium salt solution prepared in a conventional manner from 228.8 parts of the amino azo compound (VI) and 69.9 parts by volume of 40% sodium nitrite solution at 0 to 5 °C. After coupling had ended, the resulting dye of formula (VII) 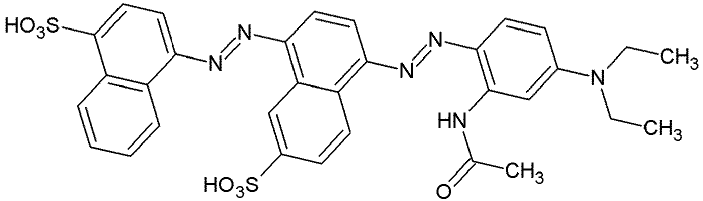 was salted out with sodium chloride, filtered off and dried at 50 °C at reduced pressure. On wool and especially on polyamide fibers it produces blue dyeings having good light and wet fastness properties with an absorption maximum of λₘₐₓ = 599 nm as measured via UV-VIS spectrometry.

### Preparation Examples 2 to 58

The following table lists compounds of formula (I) prepared according to the method described in Preparation Example 1 by using the corresponding starting materials. These compounds provide blue dyeings on polyamide fibers and wool with very good light and wet fastness.

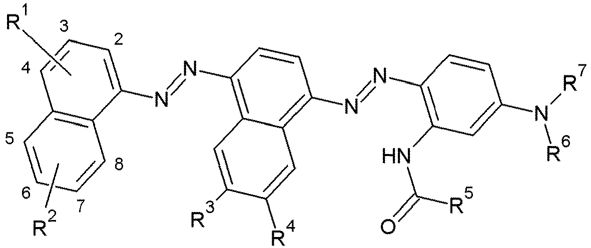

| **Example** | **R¹** | **R²** | **R³** | **R⁴** | **R⁵** | **R⁶** | **R⁷** | **λ_{max, absorption}** |
|---|---|---|---|---|---|---|---|---|
| 2 | 4-SO₃H | H | H | -SO₃H | -CH₃ | -CH₂CH₃ | -CH₂CH₃ | 600 |
| 3 | 4-SO₃H | H | -SO₃H | H | -CH₃ | -CH₂CH₂CH₂CH₃ | -CH₂CH₂CH₂CH₃ | 604 |
| 4 | 4-SO₃H | H | H | -SO₃H | -CH₃ | -CH₂CH₂CH₂CH₃ | -CH₂CH₂CH₂CH₃ | 604 |
| 5 | 4-SO₃H | H | -SO₃H | H | -CH₃ | -(CH₂)₅CH₃ | -(CH₂)₅CH₃ | 606 |
| 6 | 4-SO₃H | H | H | -SO₃H | -CH₃ | -(CH₂)₅CH₃ | -(CH₂)₅CH₃ | 605 |
| 7 | 4-SO₃H | H | -SO₃H | H | -CH₃ | -(CH₂)₄CH₃ | -(CH₂)₄CH₃ | 604 |
| 8 | 4-SO₃H | H | H | -SO₃H | -CH₃ | -(CH₂)₄CH₃ | -(CH₂)₄CH₃ | 604 |
| 9 | 4-SO₃H | H | -SO₃H | H | -CH₃ | -CH₂CH₂CH₃ | -CH₂CH₂CH₃ | 602 |
| 10 | 4-SO₃H | H | H | -SO₃H | -CH₃ | -CH₂CH₂CH₃ | -CH₂CH₂CH₃ | 603 |
| 11 | 4-SO₃H | H | -SO₃H | H | -CH₂CH₃ | -CH₂CH₂CH₂CH₃ | -CH₂CH₂CH₂CH₃ | 605 |
| 12 | 4-SO₃H | H | H | -SO₃H | -CH₂CH₃ | -CH₂CH₂CH₂CH₃ | -CH₂CH₂CH₂CH₃ | 604 |
| 13 | 4-SO₃H | H | H | -SO₃H | -CH₃ | -CH₂CH₂OH | -CH₂CH₂OH | 601 |
| 14 | 4-SO₃H | H | H | -SO₃H | -CH₂CH₃ | -CH₂CH₂OH | -CH₂CH₂OH | 600 |
| 15 | 4-SO₃H | H | -SO₃H | H | -CH₃ | -CH₂CH₂OH | -CH₂CH₂CN | 599 |
| 16 | 4-SO₃H | H | -SO₃H | H | -CH₃ | -CH₂CH(CH₃)₂ | -CH₂CH(CH₃)₂ | 604 |
| 17 | H | 7-SO₃H | H | -SO₃H | -CH₃ | -CH₂CH₂CH₂CH₃ | -CH₂CH₂CH₂CH₃ | 589 |
| 18 | H | 7-SO₃H | H | -SO₃H | -CH₃ | -CH₂CH₃ | -CH₂CH₃ | 590 |
| 19 | H | 7-SO₃H | -SO₃H | H | -CH₃ | -CH₂CH₂CH₂CH₃ | -CH₂CH₂CH₂CH₃ | 590 |
| 20 | H | 7-SO₃H | H | -SO₃H | -CH₃ | -CH₂CH₂CH₂CH₃ | -CH₂CH₂CH₂CH₃ | 589 |
| 21 | H | 7-SO₃H | -SO₃H | H | -CH₃ | -(CH₂)₅CH₃ | -(CH₂)₅CH₃ | 588 |
| 22 | H | 7-SO₃H | H | -SO₃H | -CH₃ | -(CH₂)₅CH₃ | -(CH₂)₅CH₃ | 588 |
| 23 | H | 7-SO₃H | H | -SO₃H | -CH₃ | -CH₂CH₂CH₃ | -CH₂CH₂CH₃ | 587 |
| 24 | H | 7-SO₃H | -SO₃H | H | -CH₂CH₃ | -CH₂CH₂CH₂CH₃ | -CH₂CH₂CH₂CH₃ | 589 |
| 25 | H | 7-SO₃H | -SO₃H | H | -CH₃ | -CH₂CH₂OH | -CH₂CH₂CN | 586 |
| 26 | H | 5-SO₃H | H | -SO₃H | -CH₃ | -CH₂CH₃ | -CH₂CH₃ | 598 |
| 27 | H | 5-SO₃H | -SO₃H | H | -CH₂CH₃ | -CH₂CH₃ | -CH₂CH₃ | 599 |
| 28 | H | 5-SO₃H | -SO₃H | H | -CH₃ | -CH₂CH₂CH₂CH₃ | -CH₂CH₂CH₂CH₃ | 602 |
| 29 | H | 5-SO₃H | H | -SO₃H | -CH₃ | -CH₂CH₂CH₂CH₃ | -CH₂CH₂CH₂CH₃ | 602 |
| 30 | H | 5-SO₃H | -SO₃H | H | -CH₃ | -(CH₂)₅CH₃ | -(CH₂)₅CH₃ | 603 |
| 31 | H | 5-SO₃H | H | -SO₃H | -CH₃ | -(CH₂)₅CH₃ | -(CH₂)₅CH₃ | 604 |
| 32 | H | 5-SO₃H | H | -SO₃H | -CH₃ | -(CH₂)₄CH₃ | -(CH₂)₄CH₃ | 604 |
| 33 | H | 5-SO₃H | -SO₃H | H | -CH₃ | -CH₂CH₂CH₃ | -CH₂CH₂CH₃ | 601 |
| 34 | H | 5-SO₃H | H | -SO₃H | -CH₃ | -CH₂CH₂CH₃ | -CH₂CH₂CH₃ | 602 |
| 35 | H | 5-SO₃H | -SO₃H | H | -CH₂CH₃ | -CH₂CH₂CH₂CH₃ | -CH₂CH₂CH₂CH₃ | 603 |
| 36 | H | 5-SO₃H | H | -SO₃H | -CH₃ | -CH₂CH₂OH | -CH₂CH₂OH | 605 |
| 37 | H | 5-SO₃H | H | -SO₃H | -CH₂CH₃ | -CH₂CH₂OH | -CH₂CH₂OH | 604 |
| 38 | H | 5-SO₃H | -SO₃H | H | -CH₃ | -CH₂CH₂OH | -CH₂CH₂CN | 601 |
| 39 | H | 5-SO₃H | -SO₃H | H | -CH₃ | -CH₂CH(CH₃)₂ | -CH₂CH(CH₃)₂ | 603 |
| 40 | 4-SO₃H | 6-SO₃H | H | H | -CH₃ | -CH₂CH₃ | -CH₂CH₃ | 599 |
| 41 | 4-SO₃H | 6-SO₃H | H | H | -CH₃ | -CH₂CH₂CH₂CH₃ | -CH₂CH₂CH₂CH₃ | 602 |
| 42 | 4-SO₃H | 6-SO₃H | H | H | -CH₃ | -(CH₂)₅CH₃ | -(CH₂)₅CH₃ | 604 |
| 43 | 4-SO₃H | 6-SO₃H | H | H | -CH₃ | -(CH₂)₄CH₃ | -(CH₂)₄CH₃ | 604 |
| 44 | 4-SO₃H | 6-SO₃H | H | H | -CH₃ | -CH₂CH₂CH₃ | -CH₂CH₂CH₃ | 603 |
| 45 | 4-SO₃H | 6-SO₃H | H | H | -CH₂CH₃ | -CH₂CH₂CH₂CH₃ | -CH₂CH₂CH₂CH₃ | 605 |
| 46 | 4-SO₃H | 6-SO₃H | H | H | -CH₃ | -CH₂CH₂OH | -CH₂CH₂OH | 603 |
| 47 | 4-SO₃H | 6-SO₃H | H | H | -CH₃ | -CH₂CH₂OH | -CH₂CH₂CN | 601 |
| 48 | 4-SO₃H | 6-SO₃H | H | H | -CH₃ | -CH₂CH(CH₃)₂ | -CH₂CH(CH₃)₂ | 604 |
| 49 | H | 6-SO₃H | -SO₃H | H | -CH₂CH₃ | -CH₂CH₃ | -CH₂CH₃ | 589 |
| 50 | H | 6-SO₃H | -SO₃H | H | -CH₃ | -CH₂CH₂CH₂CH₃ | -CH₂CH₂CH₂CH₃ | 590 |
| 51 | H | 6-SO₃H | H | -SO₃H | -CH₃ | -CH₂CH₂CH₂CH₃ | -CH₂CH₂CH₂CH₃ | 590 |
| 52 | H | 6-SO₃H | -SO₃H | H | -CH₃ | -(CH₂)₅CH₃ | -(CH₂)₅CH₃ | 589 |
| 53 | H | 6-SO₃H | H | -SO₃H | -CH₃ | -CH₂CH₂CH₂CH₃ | -CH₂CH₂CH₂CH₃ | 588 |
| 54 | H | 6-SO₃H | -SO₃H | H | -CH₂CH₃ | -CH₂CH₂CH₂CH₃ | -CH₂CH₂CH₂CH₃ | 588 |
| 55 | H | 6-SO₃H | H | -SO₃H | -CH₂CH₃ | -CH₂CH₂CH₂CH₃ | -CH₂CH₂CH₂CH₃ | 587 |
| 56 | H | 6-SO₃H | H | -SO₃H | -CH₃ | -CH₂CH₂OH | -CH₂CH₂OH | 589 |
| 57 | H | 6-SO₃H | H | -SO₃H | -CH₂CH₃ | -CH₂CH₂OH | -CH₂CH₂OH | 588 |
| 58 | H | 6-SO₃H | -SO₃H | H | -CH₃ | -CH₂CH₂OH | -CH₂CH₂CN | 587 |

### Application Example A

A dye bath at 40 °C, consisting of 2,000 parts of water, 1 part of a weakly cation-active levelling agent which is based on an ethoxylated aminopropyl fatty acid amide and which has affinity for dye, 0.25 part of the dye of Preparation Example 1 and adjusted to a pH of 5 with 1 to 2 parts of 40% aqueous acetic acid is entered with 100 parts of nylon-6 fabric. After 10 minutes at 40 °C, the dye bath is heated to 98 °C at a rate of 1 °C per minute and then left at the boil for 45 to 60 minutes. Thereafter it is cooled down to 70 °C over a period of 15 minutes. The dyeing is removed from the bath, rinsed with hot and then with cold water and dried. The result obtained is a blue polyamide dyeing possessing good light and wet fastness.

### Application Example B

A dye bath at 40 °C, consisting of 2,000 parts of water, 1 part of a weakly cation-active levelling agent which is based on an ethoxylated aminopropyl fatty acid amide and which has affinity for dye, 0.3 part of the dye of Preparation Example 1 and adjusted to a pH of 5.5 with 1-2 parts of 40% acetic acid is entered with 100 parts of nylon-6,6 fabric. After 10 minutes at 40 °C, the dye bath is heated to 120 °C at a rate of 1.5 °C per minute and then left at this temperature for 15-25 minutes. Thereafter it is cooled down to 70 °C over a period of 25 minutes. The dyeing is removed from the dye bath, rinsed with hot and then with cold water and dried. The result obtained is a blue polyamide dyeing with good levelness and having good light fastness and wet fastness.

### Application Example C

A dye bath at 40 °C, consisting of 4,000 parts of water, 1 part of a weakly amphoteric levelling agent which is based on a sulfated, ethoxylated fatty acid amide and which has affinity for dye, 0.4 part of the dye of Preparation Example 1 and adjusted to pH 5 with 1-2 parts of 40% acetic acid is entered with 100 parts of wool fabric. After 10 minutes at 40 °C, the dye bath is heated to boiling at a rate of 1 °C per minute and then left at the boil for 40-60 minutes. Thereafter it is cooled down to 70 °C over a period of 20 minutes. The dyeing is removed from the bath, rinsed with hot and then with cold water and dried. The result obtained is a blue wool dyeing possessing good light fastness and wet fastness.

### Application Example D

100 parts of a woven nylon-6 material are padded with a 50 °C liquor consisting of

| | |
|---|---|
| 40 | parts of the dye of Preparation Example 1, |
| 100 | parts of urea, |
| 20 | parts of a nonionic solubilizer based on butyldiglycol, |
| 15-20 | parts of acetic acid (to adjust the p_{H} to 4), |
| 10 | parts of a weakly cation-active levelling agent which is based on an ethoxylated aminopropyl fatty acid amide and has affinity for dye, and |
| 810-815 | parts of water (to make up to 1,000 parts of padding liquor). |

The material thus impregnated is rolled up and left to dwell in a steaming chamber under saturated steam conditions at 85 to 98 °C for a period of 3 to 6 hours for fixation. The dyeing is then rinsed with hot and cold water and dried. The result obtained is a blue nylon dyeing having good levelness in the piece and good light fastness and wet fastness.

### Application Example E

A textile cut pile sheet material composed of nylon-6 and having a synthetic base fabric is padded with a liquor containing per 1,000 parts

| | |
|---|---|
| 1 | part of dye of Preparation Example 1 |
| 4 | parts of a commercially available thickener based on carob flour ether |
| 2 | parts of a nonionic ethylene oxide adduct of a higher alkylphenol |
| 1 | part of 60% acetic acid. |

This is followed by printing with a paste which contains the following components per 1,000 parts:

| | |
|---|---|
| 20 | parts of commercially available alkoxylated fatty alkylamine |
| 20 | parts of a commercially available thickener based on carob flour ether |

The print is fixed for a period of 6 minutes in saturated steam at 100 °C, rinsed and dried. The result obtained is a level-colored cover material having a blue and white pattern.

### Application Example F

A dye bath at 40 °C consisting of 2,000 parts of water, 1 part of a weakly cation-active levelling agent which is based on an ethoxylated aminopropyl fatty acid amide and has affinity for dye, 0.2 part of the red dye of Example 8 of WO 2002/46318, 1.5 parts of a commercially available preparation of C.I. Acid Yellow 236 (Nylosan Yellow F-L) and 0.5 part of the blue dye of Preparation Example 1 which is adjusted to pH 5 with 1-2 parts of 40% acetic acid is entered with 100 parts of woven nylon-6,6 fabric. After a period of 10 minutes at 40 °C, the dye bath is heated to 98 °C at a rate of 1 °C per minute and then left at the boil for 45 to 60 minutes. This is followed by cooling down to 70 °C over a period of 15 minutes. The dyeing is removed from the bath, rinsed with hot and then with cold water and dried. The result obtained is a level gray polyamide dyeing having good light fastness and wet fastness.

Application examples A to F were also carried out with dyes according to Preparation Examples 2 to 58 with equally good results.

### Application Example G

3 parts of the dye according to Preparation Example 3 are dissolved in 82 parts of demineralized water and 15 parts of diethylene glycol at 60 °C. Cooling down to room temperature gives a blue printing ink which is very highly suitable for ink jet printing on paper or polyamide and wool textiles.

Application Example G was also carried out with dyes according to preparation examples 1, 2 and 4 to 58 with equally good results.

### Application Example H

A dye bath consisting of 1,000 parts of water, 80 parts of calcined Glauber salt, 1 part of sodium nitrobenzene-3-sulfonate and 1 part of dye from Preparation Example 1 is heated to 80 °C in the course of 10 minutes. Then, 100 parts of mercerized cotton are added. This is followed by dyeing at 80 °C for a period of 5 minutes and then heating to 95 °C in the course of 15 minutes. After a period of 10 minutes at 95 °C, 3 parts of sodium carbonate are added, followed by a further 7 parts of sodium carbonate after a period of 20 minutes and another 10 parts of sodium carbonate after a period of 30 minutes at 95 °C. Dyeing is subsequently continued at 95 °C for a period of 60 minutes. The dyed material is then removed from the dye bath and rinsed in running demineralized water for 3 minutes. This is followed by two washes for a period of 10 minutes in 5,000 parts of boiling demineralized water at a time and subsequent rinsing in running demineralized water at 60 °C for a period of 3 minutes and with cold tap water for a period of one minute. Drying leaves a brilliant blue cotton dyeing having good wet fastness.

### Application Example J

0.2 parts of the dye of Preparation Example 1 are dissolved in 100 parts of hot water and the solution is cooled down to room temperature. This solution is added to 100 parts of chemically bleached sulfite pulp beaten in 2,000 parts of water in a hollander. After a period of 15 minutes of commixing the stuff is sized with resin size and aluminum sulfate in a conventional manner. Paper produced from this stuff has a blue shade with good wet fastness.

Application Examples H and J were also carried out with dyes according to Preparation Examples 2 to 58 with equally good results.

## Claims

1. A compound of formula (I), in its free acid, salt or mixed salt form 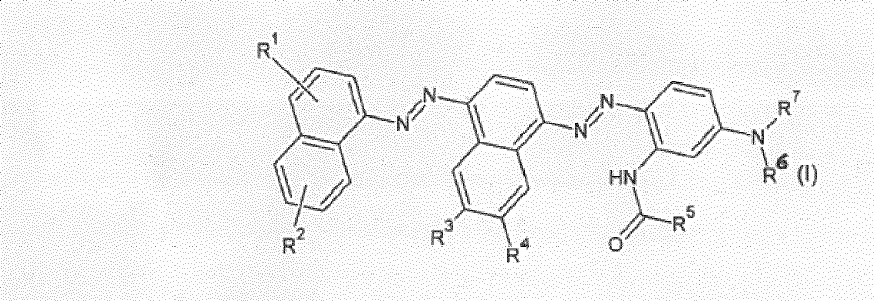 wherein
R¹ signifies hydrogen or SO₃M,
R² signifies hydrogen or SO₃M,
R³ signifies hydrogen or SO₃M,
R⁴ signifies hydrogen or SO₃M,
R⁵ signifies hydrogen, unsubstituted unbranched C₁-C₈-alkyl or unsubstituted branched C₃-C₈-alkyl, or substituted unbranched C₁-C₈-alkyl or substituted branched C₃-C₈-alkyl, substituted or unsubstituted phenyl, or unsubstituted unbranched C₁-C₁₂-alkoxy, or unsubstituted branched C₃-C₁₂-alkoxy, or substituted unbranched C₁-C₁₂-alkoxy, or substituted branched C₃-C₁₂-alkoxy, or NH₂,
R⁶ and R⁷ signify independently from each other unsubstituted unbranched C₁-C₈-alkyl or unsubstituted branched C₃-C₈-alkyl, or substituted unbranched C₁-C₈ alkyl or substituted branched C₃-C₈-alkyl, or unsubstituted aryl, or substituted -(CH₂)ₙ-aryl with n = 1, 2, 3 or 4,
wherein the substituents of the substituted aryl are selected from the group of -H, ; SO₃M, wherein M signifies a counter cation as defined below unsubstituted or substituted, linear or branched C₁₋₆ alkyl, unsubstituted or substituted, linear or
branched C₁₋₆ alkoxy;
wherein the substituents of the substituted alkyl and alkoxy groups are selected from the group consisting of heteroatoms other than hydrogen, halogen, -CN, -NH₂ or - COOM, wherein M signifies a counter cation as defined below; or wherein the substituents in R5, R6 and R7 are selected from hydroxyl group or cyano group, or from hydroxyl group and cyano group;
under the proviso that said compound of formula (I) contains at least one anionic group, preferably SO₃M group, wherein
M is selected from hydrogen cation, alkali metal cation, alkaline earth metal cation, ammonium cation and alkylammonium cation or mixtures thereof.

2. Compound according to claim 1 wherein R¹ is SO₃M and is bound to the 4-position.

3. Compound according to claim 1 or 2, wherein R² is SO₃M and is bound to the 5-, 6-, or 7-position.

4. Compound according to at least one of the claims 1 to 3, wherein the number of SO₃M groups present in the compound of formula (I) equals 1, 2, 3, or 4; preferably the number of SO₃M groups equals 2.

5. Compound according to at least one of the claims 1 to 4 wherein in the compound of formula (I) the number of SO₃M groups equals 2 and wherein R¹ and R⁴, or R¹ and R³, or R² and R⁴, or R² and R³ signify SO₃M.

6. Compound according to claim 5, wherein
R¹ and R⁴ signify SO₃M and R¹ is in the 4- position, or wherein
R¹ and R³ signify SO₃M and R¹ is in the 4- position, or wherein
R² and R⁴ signify SO₃M and R² is in the 7-position, or wherein
R² and R³ signify SO₃M and R² is in the 7-position, or wherein
R² and R⁴ signify SO₃M and R² is in the 5-position, or wherein
R² and R³ signify SO₃M and R² is in the 5-position, or wherein
R¹ and R² signify SO₃M and R¹ is in the 4-, and R² is in the 6-position, or wherein
R² and R⁴ signify SO₃M and R² is in the 6-position, or wherein
R² and R³ signify SO₃M and R² is in the 6-position.

7. Compound according to at least one of the claims 1 to 6, wherein R⁵ signifies unsubstituted unbranched C₁-C₈-alkyl, preferably methyl or ethyl.

8. Compound according to at least one of the claims 1 to 7, wherein R⁶ and R⁷ signify independently from each other unsubstituted unbranched C₁-C₆-alkyl or unsubstituted branched C₃-C₄-alkyl, or substituted unbranched C₁-C₆ alkyl or substituted branched C₃-C₄-alkyl.

9. Process for preparing a compound of formula (I) as defined in claim 1, wherein the process comprises
providing a compound of formula (II) 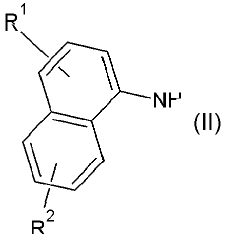
diazotizing the compound of formula (II) and coupling the diazotized compound of formula (II) onto compound of formula (III), 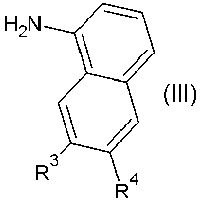
to obtain the resulting amine of formula (IV) 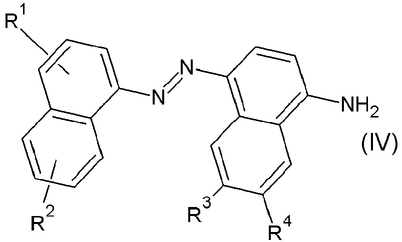
diazotizing the compound of formula (IV) and coupling the diazotized compound of formula (IV) onto compound of formula (V) 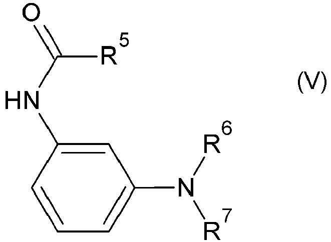
wherein the R¹ to R⁷ radicals are each as defined in at least one of claims 1 to 8.

10. Process according to claim 9, wherein the diazotization is performed at -10°C to +10°C, preferably -5°C to +10°C.

11. Use of a compound of formula (I) as defined in at least one of claims 1 to 9 or prepared according to a process according to claims 9 or 10 in a dyeing process or a printing process, preferably a process for dyeing or printing a substrate.

12. Use of a compound according to claim 11 as blue or violet component in a trichromatic dyeing or printing process.

13. Process for dyeing or printing a substrate in a trichromatic dyeing or trichromatic printing process for the manufacturing of a colored substrate, wherein the blue component used in the trichromatic dyeing or trichromatic printing process is a compound of formula (I) as defined in at least one of claims 1 to 8 or prepared according to claims 9 or 10.

14. A colored substrate comprising a compound according to at least one of claims 1 to 8, or prepared according to a process according to claims 9 or 10.

## Patentansprüche

1. Verbindung gemäß Formel (I), als freie Säure, Salz, oder in gemischter Form 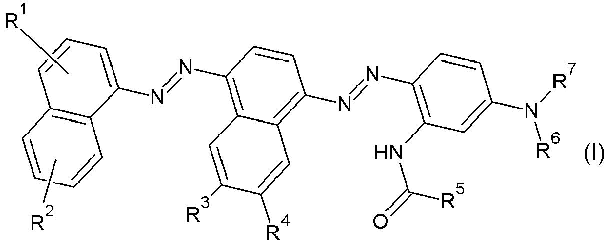 wobei
R¹ Wasserstoff oder SO₃M bedeutet,
R² Wasserstoff oder SO₃M bedeutet,
R³ Wasserstoff oder SO₃M bedeutet,
R⁴ Wasserstoff oder SO₃M bedeutet,
R⁵ Wasserstoff, unsubstituiertes, unverzweigtes C₁-C₈-Alkyl, oder unsubstituiertes, verzweigtes C₃-C₈-Alkyl, oder substituiertes, unverzweigtes C₁-C₈-Alkyl, oder substituiertes, verzweigtes C₃-C₈-Alkyl, substituiertes oder unsubstituiertes Phenyl, oder unsubstituiertes, unverzweigtes C₁-C₁₂-Alkoxy, oder unsubstituiertes, verzweigtes C₃-C₁₂-Alkoxy, oder substituiertes, unverzweigtes C₁-C₁₂-Alkoxy, oder substituiertes, verzweigtes C₃-C₁₂-Alkoxy, oder NH₂ bedeutet,
R⁶ und R⁷ unabhängig voneinander unsubstituiertes, unverzweigtes C₁-C₈-Alkyl, oder unsubstituiertes, verzweigtes C₃-C₈-Alkyl, oder substituiertes, unverzweigtes C₁-C₈-Alkyl, oder substituiertes, verzweigtes C₃-C₈-Alkyl, oder unsubstituiertes Aryl, oder substituiertes -(CH₂)ₙ-Aryl mit n = 1, 2, 3 or 4 bedeuten,
wobei die Substituenten des substituierten Aryl gewählt werden aus der Gruppe -H, - SO₃M, wobei M ein Gegenkation wie nachfolgend definiert darstellt, unsubstituiertes oder substituiertes, lineares oder verzweigtes C₁₋₆-Alkyl, unsubstituiertes oder substituiertes, lineares oder verzweigtes C₁₋₆-Alkoxy;
wobei die Substituenten der substituierten Alkyl und Alkoxy Gruppen gewählt werden aus der Gruppe bestehend aus Heteroatomen außer Wasserstoff, Halogen, -CN, -NH₂ oder -COOM, wobei M ein Gegenkation wie nachfolgend definiert darstellt; oder
wobei die Substituenten R⁵, R⁶ und R⁷ gewählt werden aus Hydroxyl oder Cyano Gruppen, oder aus Hydroxyl und Cyano Gruppen;
unter der Prämisse, dass besagte Verbindung der Formel (I) mindestens eine anionische Gruppe, vorzugsweise eine SO₃M Gruppe enthält, wobei M ausgewählt wird aus Wasserstoffkation, Alkalimetallkation, Erdalkalimetallkation, Ammoniumkation und Alkylammoniumkation, oder Mischungen davon.

2. Verbindung nach Anspruch 1, wobei R¹ SO₃M, gebunden in der 4-Position, ist.

3. Verbindung nach Anspruch 1 oder 2, wobei R² SO₃M, gebunden in der 5-, 6-, oder 7-Position, ist.

4. Verbindung nach mindestens einem der Ansprüche 1 bis 3, wobei die Zahl der SO₃M Gruppen in der Verbindung der Formel (I) 1, 2, 3 oder 4 ist; vorzugsweise ist die Zahl der SO₃M Gruppen 2.

5. Verbindung nach mindestens einem der Ansprüche 1 bis 4, wobei die Zahl der SO₃M Gruppen in der Verbindung der Formel (I) 2 ist, und wobei R¹ und R⁴, oder R¹ und R³, oder R² und R⁴, oder R² und R³ SO₃M bedeuten.

6. Verbindung nach Anspruch 5, wobei
R¹ und R⁴ SO₃M bedeuten, und R¹ in der 4-Position ist, oder wobei
R¹ und R³ SO₃M bedeuten, und R¹ in der 4-Position ist, oder wobei
R² und R⁴ SO₃M bedeuten, und R² in der 7-Position ist, oder wobei
R² und R³ SO₃M bedeuten, und R² in der 7-Position ist, oder wobei
R² und R⁴ SO₃M bedeuten, und R² in der 5-Position ist, oder wobei
R² und R³ SO₃M bedeuten, und R² in der 5-Position ist, oder wobei
R¹ und R² SO₃M bedeuten, und R¹ in der 4-, und R² in der 6-Position ist, oder wobei
R² und R⁴ SO₃M bedeuten, und R² in der 6-Position ist, oder wobei
R² und R³ SO₃M bedeuten, und R² in der 6-Position ist.

7. Verbindung nach mindestens einem der Ansprüche 1 bis 6, wobei R⁵ unsubstituiertes, unverzweigtes C₁-C₈-Alkyl bedeutet, bevorzugt Methyl oder Ethyl.

8. Verbindung nach mindestens einem der Ansprüche 1 bis 7, wobei R⁶ und R⁷ unabhängig voneinander unsubstituiertes, unverzweigtes C₁-C₆-Alkyl, oder unsubstituiertes, verzweigtes C₃-C₄-Alkyl, oder substituiertes, unverzweigtes C₁-C₆-Alkyl, oder substituiertes, verzweigtes C₃-C₄-Alkyl bedeuten.

9. Verfahren zur Herstellung einer Verbindung der Formel (I) wie in Anspruch 1 definiert, wobei das Verfahren aufweist, eine Verbindung der Formel (II) bereitzustellen, 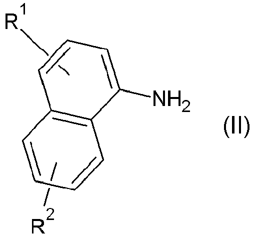 die Verbindung der Formel (II) zu diazotieren, und die diazotierte Verbindung der Formel (II) mit einer Verbindung der Formel (III) zu kuppeln, 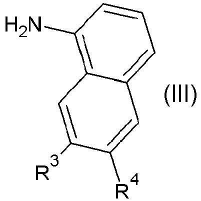 um das resultierende Amin der Formel (IV) zu erhalten, 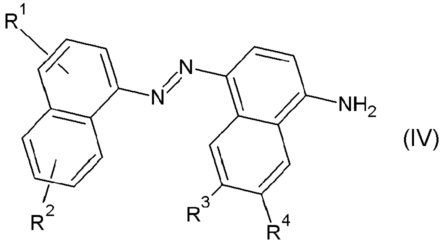 die Verbindung der Formel (IV) zu diazotieren, und die diazotierte Verbindung der Formel (IV) mit einer Verbindung der Formel (V) zu kuppeln, 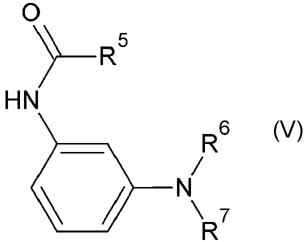 wobei die Substituenten R¹ bis R⁷ sind, wie in mindestens einem der Ansprüche 1 bis 8 definiert.

10. Verfahren nach Anspruch 9, wobei die Diazotierung bei -10°C bis +10°C durchgeführt wird, vorzugsweise bei -5°C bis +10°C.

11. Verwendung einer Verbindung der Formel (I), definiert in mindestens einem der Ansprüche 1 bis 9, oder hergestellt gemäß dem Verfahren nach Anspruch 9 oder 10, in einem Färbeprozess oder Druckprozess, vorzugsweise einem Prozess zur Färbung oder Bedruckung eines Substrats.

12. Verwendung einer Verbindung nach Anspruch 11 als blaue oder violette Komponente in einem trichromatischen Färbe- oder Druckprozess.

13. Verfahren zur Färbung oder Bedruckung eines Substrats in einem trichromatischen Färbe- oder Druckprozess zur Herstellung eines farbigen Subtrats, wobei die blaue Komponente in dem trichromatischen Färbe- oder Druckprozess eine Verbindung der Formel (I) ist, definiert in mindestens einem der Ansprüche 1 bis 8, oder hergestellt nach Anspruch 9 oder 10.

14. Farbiges Substrat aufweisend eine Verbindung nach mindestens einem der Ansprüche 1 bis 8, oder hergestellt in einem Verfahren gemäß Anspruch 9 oder 10.

## Revendications

1. Composé de formule (I), sous sa forme d'acide libre, de sel ou de sel mixte 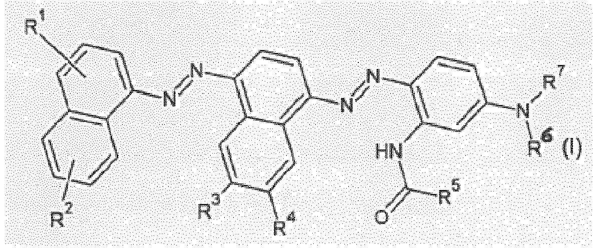 dans lequel
R¹ représente un hydrogène ou SO₃M,
R² représente un hydrogène ou SO₃M,
R³ représente un hydrogène ou SO₃M,
R⁴ représente un hydrogène ou SO₃M,
R⁵ représente un hydrogène, un alkyle en C₁ à C₈ non ramifié non substitué ou un alkyle en C₃ à C₈ ramifié non substitué, ou un alkyle en C₁ à C₈ non ramifié substitué ou un alkyle en C₃ à C₈ ramifié substitué, un phényle substitué ou non substitué, ou un alcoxy en C₁ à C₁₂ non ramifié non substitué, ou un alcoxy en C₃ à C₁₂ ramifié non substitué, ou un alcoxy en C₁ à C₁₂ non ramifié substitué, ou un alcoxy en C₃ à C₁₂ ramifié substitué, ou NH₂,
R⁶ et R⁷ représentent indépendamment l'un de l'autre un alkyle en C₁ à C₈ non ramifié non substitué ou un alkyle en C₃ à C₈ ramifié non substitué, ou un alkyle en C₁ à C₈ non ramifié substitué ou un alkyle en C₃ à C₈ ramifié substitué, ou un aryle non substitué, ou -(CH₂)ₙ-aryle substitué avec n = 1, 2, 3 ou 4,
dans lequel les substituants de l'aryle substitué sont choisis parmi le groupe constitué de -H, -SO₃M, où M représente un contre-cation tel que défini ci-dessous, un alkyle en C₁ à C₆ linéaire ou ramifié, non substitué ou substitué, un alcoxy en C₁ à C₆ linéaire ou ramifié, non substitué ou substitué ;
dans lequel les substituants des groupes alkyle et alcoxy substitués sont choisis parmi le groupe constitué d'hétéroatomes autres que l'hydrogène, un halogène, -CN, -NH₂ ou-COOM, où M représente un contre-cation tel que défini ci-dessous ; ou
dans lequel les substituants dans R⁵, R⁶ et R⁷ sont choisis parmi un groupe hydroxyle ou un groupe cyano, ou parmi un groupe hydroxyle et un groupe cyano ;
à condition que ledit composé de formule (I) contienne au moins un groupe anionique, préférentiellement un groupe SO₃M, où
M est choisi parmi un cation d'hydrogène, un cation de métal alcalin, un cation de métal alcalino-terreux, un cation ammonium et un cation alkylammonium ou des mélanges de ceux-ci.

2. Composé selon la revendication 1 dans lequel R¹ est SO₃M et est lié en position 4.

3. Composé selon la revendication 1 ou 2, dans lequel R² est SO₃M et est lié en position 5, 6 ou 7.

4. Composé selon au moins l'une des revendications 1 à 3, dans lequel le nombre de groupes SO₃M présents dans le composé de formule (I) est égal à 1, 2, 3 ou 4; préférentiellement le nombre de groupes SO₃M est égal à 2.

5. Composé selon au moins l'une des revendications 1 à 4, dans lequel dans le composé de formule (I) le nombre de groupes SO₃M est égal à 2 et dans lequel R¹ et R⁴, ou R¹ et R³, ou R² et R⁴, ou R² et R³ représentent SO₃M.

6. Composé selon la revendication 5, dans lequel
R¹ et R⁴ représentent SO₃M et R¹ est en position 4, ou dans lequel
R¹ et R³ représentent SO₃M et R¹ est en position 4, ou dans lequel
R² et R⁴ représentent SO₃M et R² est en position 7, ou dans lequel
R² et R³ représentent SO₃M et R² est en position 7, ou dans lequel
R² et R⁴ représentent SO₃M et R² est en position 5, ou dans lequel
R² et R³ représentent SO₃M et R² est en position 5, ou dans lequel
R¹ et R² représentent SO₃M et R¹ est en position 4, et R² est en position 6, ou dans lequel
R² et R⁴ représentent SO₃M et R² est en position 6, ou dans lequel R² et R³ représentent SO₃M et R² est en position 6.

7. Composé selon au moins l'une des revendications 1 à 6, dans lequel R⁵ représente un alkyle en C₁ à C₈ non ramifié non substitué, préférentiellement un méthyle ou un éthyle.

8. Composé selon au moins l'une des revendications 1 à 7 dans lequel R⁶ et R⁷ représentent indépendamment l'un de l'autre un alkyle en C₁ à C₆ non ramifié non substitué ou un alkyle en C₃ à C₄ ramifié non substitué, ou un alkyle en C₁ à C₆ non ramifié substitué ou un alkyle en C₃ à C₄ ramifié substitué.

9. Procédé de préparation d'un composé de formule (I) tel que défini dans la revendication 1, dans lequel le procédé comprend la fourniture d'un composé de formule (II) 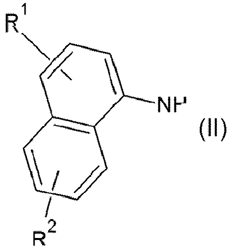 la diazotation du composé de formule (II) et la copulation du composé diazoté de formule (II) sur le composé de formule (III), 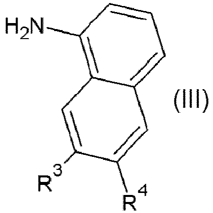 afin d'obtenir l'amine résultante de formule (IV) 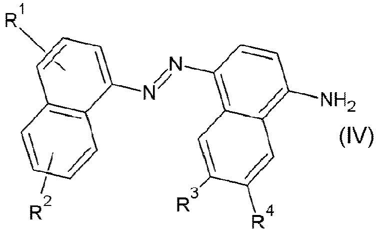 la diazotation du composé de formule (IV) et la copulation du composé diazoté de formule (IV) sur le composé de formule (V) 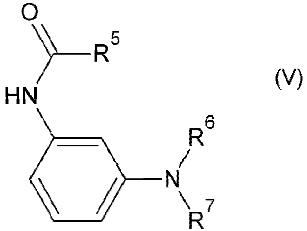 dans lequel les radicaux R¹ à R⁷ sont chacun tels que définis dans au moins l'une des revendications 1 à 8.

10. Procédé selon la revendication 9, dans lequel la diazotation est réalisée à une température de -10 °C à +10 °C, préférentiellement de -5 °C à +10 °C.

11. Utilisation d'un composé de formule (I) tel que défini dans au moins l'une des revendications 1 à 9 ou préparé selon un procédé tel que revendiqué dans les revendications 9 ou 10 dans un procédé de teinture ou un procédé d'impression, préférentiellement un procédé pour la teinture ou l'impression d'un substrat.

12. Utilisation d'un composé selon la revendication 11 en tant que composant bleu ou violet dans un procédé de teinture ou d'impression trichromatique.

13. Procédé pour la teinture ou l'impression d'un substrat dans un procédé de teinture trichromatique ou d'impression trichromatique pour la fabrication d'un substrat coloré, dans lequel le composant bleu utilisé dans le procédé de teinture trichromatique ou d'impression trichromatique est un composé de formule (I) tel que défini dans au moins l'une des revendications 1 à 8 ou préparé selon les revendications 9 ou 10.

14. Substrat coloré comprenant un composé selon au moins l'une des revendications 1 à 8, ou préparé selon un procédé tel que revendiqué dans les revendications 9 ou 10.
